Europäisches Patentamt

European Patent Office

Office européen des brevets

(11) Publication number: **0 458 258 A1**

# EUROPEAN PATENT APPLICATION

(21) Application number: **91108170.1**

(22) Date of filing: **21.05.91**

(51) Int. Cl.⁵: **A61C 8/00, A61F 2/00**

(30) Priority: **22.05.90 IL 94477**

(43) Date of publication of application:
**27.11.91 Bulletin 91/48**

(84) Designated Contracting States:
**AT BE CH DE DK FR GB IT LI NL SE**

(71) Applicant: **IMPLADRILL LIMITED**
**Segula Industrial Zone, P.O. Box 4584**
**Petah Tikva(IL)**

(72) Inventor: **Perez, Michael**
**Kibbutz Kfar Glickson**
**Doar-Na Menashe 37815(IL)**
Inventor: **Levy, Haim**
**50 Hansiim St.**
**Petah Tikva(IL)**

(74) Representative: **Prato, Roberto et al**
**STUDIO TORTA Società Semplice Via Viotti 9**
**I-10121 Torino(IT)**

(54) Device for performing surgical implants.

(57) A device (10) for performing surgical implants constitutes an implant of the screw type and comprises a portion (13) which constitutes a drill-bit (14-14';15-15'),adapted for perforating the bone in which the implant is to be seated.

FIG. 1

EP 0 458 258 A1

## Field of The Invention

This application refers to a device for performing surgical implants. Particular reference is made herein to dental implants, but the device according to the invention may be used for any implants in any bones of the human body.

## BACKGROUND OF THE INVENTION

Implants are means for anchoring protheses to bones. According to the state of the art, implants may have a round or a flat cross-section, and those having a round cross-section may be in the shape of pins or screws. This invention relates specifically to implants of the screw type, which are the most widely used in modern surgical techniques. The method followed for applying these latter involves drilling a seat in the bone for the implant, creating therein a screw thread and screwing the implant into the seat. After a certain period of time, required for osteointegration, the implant is exposed and thereafter the desired fixed or removable prothesis may be mounted therein.

The material used for the implants is usually a titanium alloy for surgical uses, which is designated as Ti-4V-A1-ELI. Hydroxyapatit or titanium coated with hydroxyapatit may also be used. Titanium has been proved to be the best material for producing osteointegration, which is critical for the success of the implant. Once the implant has been inserted into the bone under sterile conditions and has remained therein for a number of months, from 3 to 6 in general, without being subjected to any load, the implant becomes directly bound to the bone. The phenomenon of osteointegration is the basis of the implant technique.

## The Prior Art

The technique for effecting implants of the screw type, according to present day art, involves an implantation process that comprises essentially six stages. In the first stage the bone is exposed by extensive removal of the periosteum to uncover the location at which the implant must be applied, which location must be free from anatomical structures such as nerves, large blood vessels and sinuses. In the second stage a first perforation is made by means of a thin drill without any internal cooling. In the third stage the perforation is enlarged by means of a drill having an intermediate size, with internal cooling. In the fourth stage the perforation is further enlarged to its final diameter by means of a drill having the appropriate size, with internal cooling. In the fifth stage, the perforation is threaded by means of a hand-actuated screw bit, without internal cooling. In the last stage the hand-actuated bit is removed and the implant is screwed in place mechanically or by means of a hand-operated key. The speed of the drill in the drilling stages is quite high and may reach 4000-5000 r.p.m.

It is seen that the said process has considerable drawbacks. Firstly, it is complex and lengthy. Then, the high drilling speed generates excessive heat and requires cooling by means of a stream of physiological solution. The cooling liquid necessarily removes from the perforation the bone chips which are produced and which, if they had remained in place, would have played a useful role as filling material and for accelerating the process of osteointegration. The duration of the process increases the danger of infection, in spite of all the precautions that are taken. The several stages of the implantation require a sophisticated technique and cause considerable discomfort to the patient; and further, make the treatment costly, all the more so in view of the complexity of the equipment required.

It is an object of this invention to eliminate the aforesaid drawbacks and to improve the implantation process, rendering it faster and easier and reducing the discomfort to the patient.

It is a further object of this invention considerably to reduce the cost of the equipment required for the implantation.

It is a still further object of the invention to permit better control of the implantation operations on the part of the operating surgeon.

Other objects and advantages of the invention will become apparent as the description proceeds.

## SUMMARY OF THE INVENTION

The device according to the invention is a combined implant-drill tool, viz it is characterized in that it constitutes an implant of the screw type and comprises a portion which constitutes a drill-bit, adapted for perforating the bone in which the implant is to be seated.

More specifically, the device according to the invention comprises a body, a perforating or drill-bit portion, means for imparting to the device a rotary motion to perforate the bone and insert the device therein, and means for receiving prothesis elements.

Preferably, recesses are provided in the body of the device for facilitating anchorage thereof to the bone through the processes of osteointegration.

Preferably, the device is made of titanium or hydroxyapatit or titanium coated with hydroxyapatit.

In an embodiment of the invention, the means for receiving prothesis elements comprise an axial perforation formed in the body of the device, having therein a female screw thread or other means for attaching a prothesis thereto.

In another embodiment of the invention, said means comprise a projection provided with a screw thread or other means for attaching a prothesis thereto.

Preferably, the means for imparting to the device a rotary motion may comprise a seat of any desired kind for a key, or other coupling means, defined e.g. in the body of the device, for receiving a preferably hand-actuated tool in engaging relationship.

Other characteristics of the invention will appear from the description of a preferred embodiment thereof, with reference to the appended drawings, wherein:

## BRIEF DESCRIPTION OF THE DRAWINGS

- Fig. 1 is a perspective view of a device according to an embodiment of the invention;
- Fig. 2 is a side view of the same;
- Fig. 3 is an axial cross-section of the same;
- Fig. 4 is an end view of the same device, seen from the front, viz. the end which constitutes a drill-tool;
- Fig. 5 is an end view of the same device, seen from the rear, viz. from the end opposite to that shown in Fig. 4; and
- Figs. 6, 7, 8 and 9 are views, corresponding to Figs. 1, 2, 3 and 5 respectively, of device according to another embodiment of the invention.

## DETAILED DESCRIPTION OF PREFERRED EMBODIMENTS

With reference to the drawings, numeral 10 generally designates a device according to an embodiment of the invention, which is at the same time an implant and a drill-tool. The device comprises a body or externally cylindrical portion 11, which, in the embodiment herein illustrated, is provided with a serration 12 to facilitate its anchorage in the bone by the process of osteointegration. The device has an end 13, which may be called the front end and which is shaped as a perforating bit. Front end 13 has cutting edges 14-14' and 15-15', a point 16 and a serration 16', the purpose of which is to provide an anchorage of the implant after it has been filled with bone tissue.

The body 11 of the device is provided with an internal cavity 20, which has a female screw thread 21, which serves as a seat for the prothesis elements which will be applied later on. To the rear of cavity 20 there is provided a seat, which in this embodiment is another cavity 22, which has a polygonal, illustrated as a hexagonal, shape, for receiving in operative engagement a tool, preferably a hand actuated key, for rotating the device when it is inserted into the bone. Of course, other means for providing engagement of the device with a key or other tool, for the purpose of actuating the device, could be provided in this or other embodiments of the invention.

It should be understood that the invention may be carried out in different ways, and the device may have different configurations, as long as it is so structured as to serve as an implant to which the desired prothesis elements may be applied, according to the needs of the particular case, and comprises a portion which is structured as a perforating or drill-bit and is therefore capable of perforating the bone when the device is actuated for rotary and advancing motion. The essence of the invention is, in fact, the combination in a single element of the implant and the drill tool.

The use of the device, viz. the process by which the implant is effected, is extremely simple and actually involves only three stages: exposing the bone portion in which the perforation is to be made, performing an initial perforation and screwing-in the combined implant and drill tool, preferably by hand.

The initial perforation is carried out with a thin drill bit and its purpose is to prevent sliding of the implant at the beginning of its introduction. In other words, it serves to prepare a recess or seat for facilitating the introduction of the implant. It may, however, be omitted in some cases, e.g. if the bone is particularly soft or if the implant is carried out in a cavity after extraction of a tooth. The diameter of the drill bit for said initial perforation is commonly 0.5 mm or thereabout. The drill is usually not provided with internal cooling, though it may, if so desired but not necessarily, be cooled externally. It is actuated electrically or pneumatically and is a part of the conventional dental surgery equipment. The perforation is carried out at a low rotary speed, e.g., not more than 20 - 30 r.p.m, and therefore no cooling is required. Once the implant is in place, the cavities thereof are closed by means of a plastic stopper introduced in cavity 22 or by any other suitable means. The implant remains in place until osteointegration has taken place and the remaining phases of the treatment, until the application of the final prothesis, are carried out in the usual way, as with conventional implants.

It is easily understood that, in this way, no excessive heat is generated. It is also clear that the operation is simpler and faster than the known techniques and involves much less discomfort to the patient. Furthermore, the bone fragments which are produced in the perforation remain in place, since no cooling liquid flows through the perforation, and they serve as useful filling material, the presence of which greatly accelerates the process of welding of the implant to the bone.

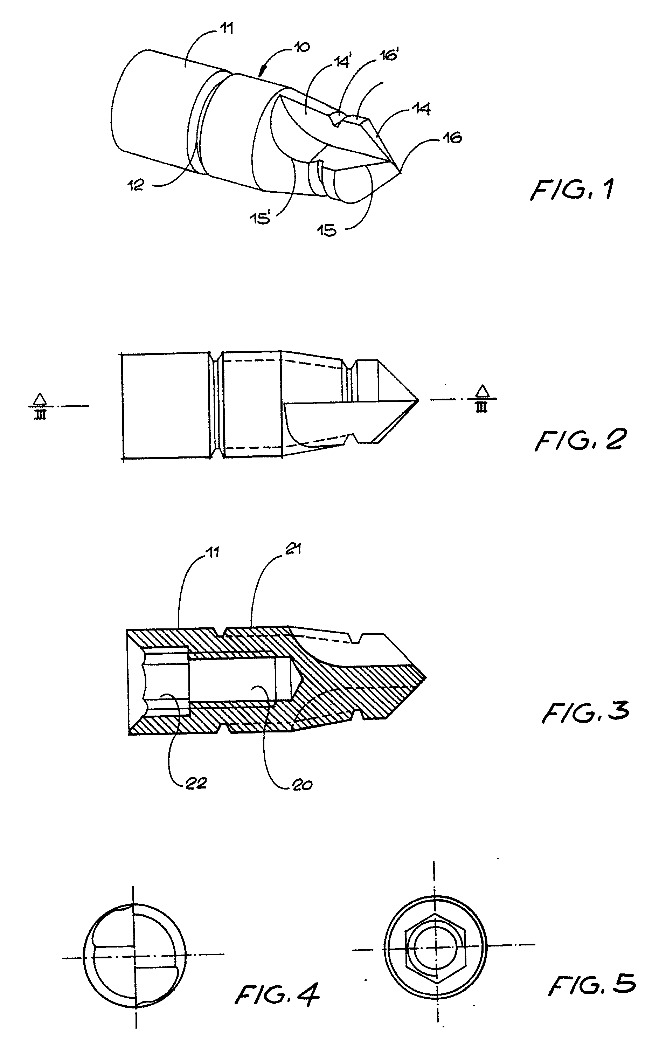

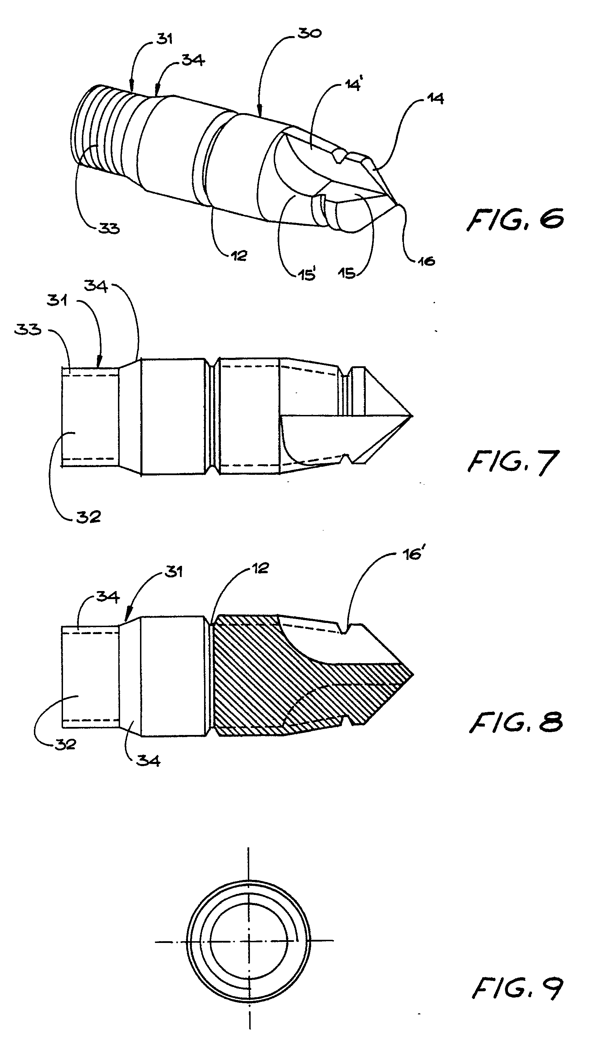

Another preferred embodiment of the device is illustrated in Figs. 6 to 9. As it is seen, the device 30 therein illustrated is essentially similar to tthat of the first embodiment described, except as to the means for receiving the prothesis and the means for turning it by a hand-actuated tool. The parts which are unchanged with respect to Figs. 1 to 5 are designated by the same numerals and need not be further described. The rear portion of the device, generally indicated at 31, comprises a projection 32 perovided with a male screw thread 33 and preferably connected to the central portion of the device by a preferably conical shoulder 34. After the implant has been effected and completed, the prothesis is attached thereto by screwing it onto projection 32 until it firmly engages shoulder 34. Of course, the prothesis must be shaped accordingly, exactly to match screw thread 33 and shoulder 34.

The advantages of this embodiment are several. Since the projection 32 is exposed to the eye, it is possible to examine it and check, e.g., that it is clean and free of extraneous matter and of anything that may interfere with a perfect engagement and attachment of the prothesis; and if any such matter should be present, it is easy to eliminate it. Secondly, this embodiment results in an implant that may be shorter, since no cavity must be provided in its body. Thirdly, the diameter of the thread engagement between implant and prothesis may be increased, all other things being equal, thereby strengtening the prothesis and rendering it more stable. Furthly, the projection 32 may and generally will be covered with a cap, e.g. a plastic cap, and this will be exposed, showing the exact location of the implant and making it possible to feel its position by touch. Finally, the shoulder 34 absorbs lateral forces and moments and produces a better engagement of the implant with the prothesis.

Means are provided ,in the implant according to this embodiment as well, for inserting a key to rotate it. Thus a suitable, e.g. square or hexagonal cavity or a seat for a Phillips screw-driver could be provided in the center of projection 32. Such means are not shown inn the drawing as they are conventional and can easily be provided by persons skilled in the art.

Thanks to the invention, the equipment required for carrying out implants is greatly simplified and made much more economical. The invention also permits a great saving of time in the implanting operation. After exposing the bone portion into which the implant is to be inserted, 2 minutes are sufficient, instead of the 15 required according to the common practice of the prior art. Thus, if the operation is carried out immediately after extraction of a tooth, it may be completed in as little as 2 minutes, while implantations which require removal of the periosteum are completed in about 10 minutes for the first implantation and 2 minutes for any successive one. The shortening of the operation of course reduces the chances of infection and greatly decreases the discomfort of the patient. It is also easier to carry out an implantation by using the invention than by the prior art processes.

The invention is applicable with success to a great variety of implantations in any kind of bones and for any kind of patient. It also shortens the period which must pass after the introduction of the implant until the further operations required for completing the implantation may be carried out.

The implant according to the invention can be manufactured in a variety of sizes, to cover all practical needs. E.g., its length may vary from 4 to 22 mm and its diameter from 2 to 5.5. mm, but these sizes should not be considered as limitative, since the invention involves no such limitation. The device according to the invention is adapted for the application of any desired protheses, be they removable, semi-removable or fixed.

While two preferred embodiments of the invention have been described by way of example, it will be understood that the invention is not limited thereto and may be carried out in a number of different ways by skilled persons without departing from its spirit and from the scope of the appended claims.

## Claims

1. Device for performing surgical implants, characterized in that it constitutes an implant of the screw type and comprises a portion which constitutes a drill-bit, adapted for perforating the bone in which the implant is to be seated.

2. Device according to claim 1, comprising a body, a perforating or drill-bit portion, means for imparting to the device a rotary motion to perforate the bone and insert the device therein, and means for receiving prothesis elements.

3. Device according to claim 1 or 2, comprising recesses in the body thereof for facilitating anchorage thereof to the bone through the processes of osteointegration.

4. Device according to any one of the foregoing claims, characterized in that it is made of titanium or hydroxyapatit or titanium coated with hydroxyapatit.

5. Device according to any one of the foregoing claims, wherein the means for receiving prothesis elements comprise an axial perforation formed in the body of the device, having therein a female screw thread.

6. Device acccording to any one of claims 1 to 4, wherein the means for receiving prothesis elements comprise a projection formed in the body of the device, having therein a male screw thread.

7. Device according to any one of the foregoing claims, wherein the means for imparting to the device a rotary motion comprise a seat in the body of the device for receiving a key or other in engaging relationship.

8. Device according to any one of claims 1 to 6, wherein the means for imparting to the device a rotary motion comprise a projection for receiving a hand-actuated tool in engaging relationship.

9. Process for inserting surgical implants in bones, comprising the steps of providing an implant comprising a portion structured as a drill tool, exposing the bone portion in which the implantation has to be made, drilling a seat for the implant by screwing into the bone the said implant, preferably by hand operation, and leaving the said implant in place in the seat thus formed.

10. Process according to claim 10, further comprising carrying out asn initial perforation with a power operated drill having a small diameter, before screwing the implant into the bone.

FIG. 1

FIG. 2

FIG. 3

FIG. 4

FIG. 5

FIG. 6

FIG. 7

FIG. 8

FIG. 9

7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 282 789 (HANS GRAFELMANN) <br> * Column 3, lines 5-45; figure 1 * <br> – – – | 1-3,5,7,9, 10 | A 61 C 8/00 <br> A 61 F 2/00 |
| X | EP-A-0 323 559 (MONDANI) <br> * Description; figures * <br> – – – | 1,2,6, 8-10 | |
| X | EP-A-0 237 505 (NOBELPHARMA) <br> * Page 5, lines 24-28; claim 1 * <br> – – – – – | 1,4,9,10 | |

| | TECHNICAL FIELDS SEARCHED (Int. Cl.5) |
|---|---|
| | A 61 C <br> A 61 F |

The present search report has been drawn up for all claims

| Place of search | Date of completion of search | Examiner |
|---|---|---|
| The Hague | 14 August 91 | VANRUNXT J.M.A. |